(19) Europäisches Patentamt
European Patent Office
Office européen des brevets

(11) Veröffentlichungsnummer: **0 291 663**
**A2**

(12) # EUROPÄISCHE PATENTANMELDUNG

(21) Anmeldenummer: **88104747.6**

(51) Int. Cl.⁴: **A61K 31/135**

(22) Anmeldetag: **24.03.88**

Die Bezeichnung der Erfindung wurde geändert (Richtlinien für die Prüfung im EPA, A-III, 7.3).

(30) Priorität: **25.03.87 DE 3709689**

(43) Veröffentlichungstag der Anmeldung: **23.11.88 Patentblatt 88/47**

(84) Benannte Vertragsstaaten: **AT BE CH DE FR GB IT LI LU NL SE**

(71) Anmelder: **Helopharm W. Petrik GmbH & Co.KG.**
**Waldstrasse 23-25**
**D-1000 Berlin 51(DE)**

(72) Erfinder: **Petrik, Gerd**
**Herthastrasse 11**
**D-1000 Berlin 33(DE)**

(74) Vertreter: **Vossius & Partner**
**Siebertstrasse 4 P.O. Box 86 07 67**
**D-8000 München 86(DE)**

(54) **Tert.-Butylamino-3-(phenyl-2'-phenoxy)-1-propanol-2 zur Bekämpfung von Herzarrhythmie.**

(57) Beschrieben ist tert.-Butylamino-3-(phenyl-2'-phenoxy)-1-propanol-2 und seine Salze mit Säuren, sowie diese Verbindung enthaltende Arzneimittel, die als Antiarrhythmika verwendet werden.

EP 0 291 663 A2

**Tert.-Butylamino-3-(phenyl-2'-phenoxy)-1-propanol-2 und seine Salze mit Säuren zur Anwendung als Arzneistoff"**

Aus der FR-PS 1 494 749 sind 2-Hydroxydiphenyl-Derivate bekannt, die an der phenolischen Hydroxylgruppe durch eine 2-Hydroxy-3-alkylaminopropyl-Gruppe veräthert sind. In Beispiel 5 ist das tert.-Butylamino-3-(phenyl-2'-phenoxy)-1-propanol-2 und sein Chlorhydrat sowie ein Verfahren zu seiner Herstellung beschrieben. Diese Verbindungen sollen β-Symphatholytika, d.h. β-Rezeptorenblocker, sein. Pharmakologische und toxikologische Daten sind in der Patentschrift nicht angegeben. Diese Verbindungen haben, soweit bekannt, bisher keinen Eingang in die Medizin gefunden.

Überraschenderweise wurde gefunden, daß das tert.-Butylamino-3-(phenyl-2'-phenoxy)-1-propanol 2 ("Bipranol") der Formel

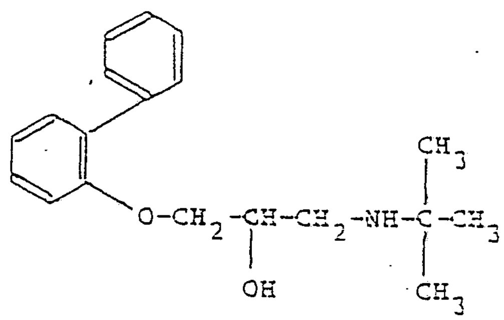

und seine Salze mit Säuren keine β-sympatholytische Wirkung, sondern eine starke antiarrhythmische Wirkung besitzen. Die elektrophysiologischen und hämodynamischen Wirkungen wurden in vitro an zwei verschiedenen isolierten Herzpräparaten und in vivo (Hund) experimentell untersucht.

In Tabelle I sind Ergebnisse pharmakologischer Untersuchungen mit Diprafenon, Propafenon und Bipranol zusammengefaßt.

Tabelle I

| Herzpräparat | Hunde-Purkinye Fasern Rate Factor von $V_{max}$ | C20APD95 µMol | Meerschweinchen-Papillarmuskel, Sf |
|---|---|---|---|
| Diprafenon | 1,19 | nicht erreicht | 4,86 |
| Propafenon | 1,08 | nicht erreicht | 1,55 |
| Bipranol | 5 | 7,2 | 9,09 |

C20 $V_{max}$: Konzentration, die $V_{max}$ bei einer Zykluslänge von 1000 mSek. um 20 % verringert.

C20 CF: Konzentration, die die Kontraktion des Papillarmuskels um 20 % verringert.

Rate Factor von $V_{max}$: (% des Kontrollwertes $V_{max}$ bei einer Zykluslänge von 2000 mSek.)/(% des Kontrollwertes $V_{max}$ bei einer Zykluslänge von 500 mSek.) bei C20$V_{max}$.

C20APD95: Die Konzentration, die das APD95 des normalen Aktionspotentials um 20 % verlängert.

Sf : Sicherheitsfaktor, errechnet als Rate Factor von $V_{max}$ × (C20 CF/C20 $V_{max}$).

Besonders bemerkenswert ist dabei der hohe Sicherheitsfaktor von 9,09 sowie der hohe Rate Factor $V_{max}$ von Bipranol im Vergleich zu Diprafenon und Propafenon. Dieser Rate Factor ist ein Indiz dafür, daß die Verbindung umso besser wirkt, je schneller die Herzfrequenz ist. Der C20APD95 Wert bedeutet, daß das Aktionspotential bei einer Konzentration von Bipranol von 7,2 µMol um 20 % verlängert wird.

Der hohe Sicherheitsfaktor zeigt, daß Bipranol keine nennenswerte negativ-inotrope Nebenwirkung hat. Demgegenüber war bei Diprafenon sowie Propafenon ein deutlich negativer inotroper Effekt nachweisbar. Im Gegensatz zu den Vergleichsverbindungen kann Bipranol und seine Salze mit Säuren offen sichtlich ohne großes Risiko zur Behandlung von Herzarrhythmien eingesetzt werden, besonders bei Patienten, bei denen die Gefahr einer Herzinsuffizienz besteht.

Dies wird auch durch in vivo Experimente bestätigt. Die antiarrhythmische Wirksamkeit der Verbindung wurde an Hunden nach H. Glücker et al., Arzneim.Forsch. (Drug Ros.) Bd. 35 (II), 9 (1985) S.1387-1393 geprüft. Die linke Koronararterie wurde mindestens 2 Stunden lang occludiert. Nach dem Auftreten konstanter ventrikulärer Arrhythmien wurden die Verbindungen intravenös injiziert. Bei einer Dosierung von 1.6 mg/kg gefolgt von 50 µg/kg/min bewirkte Bipranol eine über 90%ige Reduktion der ventrikulären Extrazystolen.

Die therapeutische Dosierung liegt wesentlich unterhalb des Wertes z.B. für Flecainid (2,4 mg/kg und 50 µg/kg/min), das zur Zeit als eines der wirksamsten Antiarrhythmika angesehen wird.
Innerhalb der therapeutischen Dosierung (0,2 bis 6,4 mg/kg) bewirkt Bipranol keine oder nur geringfügige kardiodepressive oder hämodynamische Nebenwirkungen.

Als Parameter zur Bestimmung der β-Rezeptor-blockierenden Wirkung am narkotisierten Hund dienten dP/dt als Maß für die Kontraktionskraft des Herzens, sowie cardiac output (Herzzeitvolumen). Bipranol hatte bis 3 mg/kg eine positiv-inotrope Wirkung und bis zu 30 mg/kg keine negativ-inotrope Wirkung. Im Gegensatz wirken β-Blocker immer negativ-inotrop. Auch cardiac output wurde bis zu einer Dosis von 30 mg/kg nicht beeinflußt. Bei der höchsten Dosis (30 mg/kg i.v.) starben alle Hunde. Die Beobachtungen bei dieser Dosis sind wahrscheinlich Folge einer nicht spezifischen toxischen Wirkung und beruhen nicht auf einer β-sympatolytischen Wirkung.

In in vitro-Experimenten an isolierten Herzpräparaten lag die leistungsverzögernde und damit antiarrhythmische Wirkung (C20 $V_{max}$) bei einer Dosis von 2,2 µMol Bipranol. Diese Dosierung entspricht einer antiarrhythmischen wirksamen Dosis von etwa 3 mg/kg bei in vivo Versuchen an Hunden. Am isolierten Meerschweinchen-Vorhofmuskel bewirkte Bipranol bei der höchsten Versuchskonzentration (30 µMol) lediglich eine geringe Abschwächung der positiv-inotropen Wirkung von 1 µMol Adrenalin. Diese Konzentration ist ungefähr 14 mal höher als für eine antiarrhythmische Wirkung gebraucht wird. Sie liegt wahrscheinlich im toxischen Bereich und deswegen ist diese geringe Abschwächung nicht auf eine β-sympatolytische sondern auf nicht spezifische toxische Wirkungen zurückzuführen.

Diese überraschend hohe antiarrhythmische Wirksamkeit ist nicht begleitet von einer signifikanten Toxizitätssteigerung. Dies ergibt sich aus einem Vergleich der $LD_{50}$-Werte bei Ratten, die in Tabelle II zusammengefaßt sind.

Tabelle II

| | Propafenon HCl | Bipranol HCl (mg/kg) |
|---|---|---|
| $LD_{50}$ | | |
| Ratte (iv) | 18,8 | 11 |
| Ratte (oral) | 760 | 571 |

Diese Versuchsergebnisse lassen erwarten, daß Bipranol und seine Salze mit Säuren wie auch Propafenon zur Therapie von Herzrhythmusstörungen in der Humanmedizin erfolgreich eingesetzt werden können. Im Gegensatz jedoch zu Propafenon, das zusätzlich auch stark blutdrucksenkende und negativ-inotrope Nebenwirkungen hat, zeigt Bipranol nur geringfügige oder keine hämodynamischen und negativ inotropen Nebenwirkungen, woraus sich eine erhebliche Vergrößerung der therapeutischen Breite ergibt.

Die Herstellung von Bipranol kann nach dem in der FR-PS 1 494 749 beschriebenen Verfahren aus o-(Epoxy-2,3-propoxy)-diphenyl und tert.-Butylamin erfolgen.

Gegebenenfalls wird Bipranol mit einer anorganischen oder organischen Säure in ein Salz überführt. Beispiele für geeignete Säuren sind Salzsäure, Bromwasserstoffsäure, Phosphorsäure, Schwefelsäure,

Oxalsäure, Maleinsäure, Fumarsäure, Milchsäure, Weinsäure, Äpfelsäure, Citronensäure, Salicylsäure, Adipinsäure und Benzoesäure; vgl. auch Fortschritte der Arzneimittelforschung, Bd. 10, 1966; S. 224-225, Birkhäuser Verlag, Basel und Stuttgart.

Die Salze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmakologisch verträgliche wässrige Lösungen der Salze von Bipranol durch Auflösen der freien Base in einer wässrigen Säurelösung hergestellt werden.

Bipranol und seine Salze mit Säuren können in üblicher Weise oral oder intravenös verabfolgt werden. Die Dosierung hängt vom Alter, Zustand und Gewicht des Patienten sowie von der Applikationsart ab. In der Regel beträgt die tägliche Wirkstoffdosis etwa 5 bis 75 mg/kg Körpergewicht bei oraler Gabe und etwa 1 bis 10 mg/kg Körpergewicht bei parenteraler Gabe.

Bipranol und seine Salze können in üblichen galenischen Applikationsformen fest oder flüssig angewendet werden, z.B. als Tabletten, Filmtabletten, Kapseln, Pulver, Granulate, Dragees, Suppositorien oder Lösungen. Diese werden in üblicher Weise hergestellt. Der Wirkstoff kann dabei mit den üblichen Hilfsmitteln, wie Tablettenbindern, Füllstoffen, Konservierungsmitteln, Tablettensprengmitteln, Fließreguliermitteln, Weichmachern, Netzmitteln, Dispergiermitteln, Emulgatoren, Losungsmitteln, Retardierungsmitteln und/oder Antioxidantien, verarbeitet werden; vgl. H. Sucker et al., Pharmazeutische Technologie, Thieme-Verlag, Stuttgart, 1978. Die so erhaltenen Applikationsformen enthalten den Wirkstoff normalerweise in einer Menge von 1 bis 99 Gew.-%.

Die Beispiele erläutern die Erfindung.

**Beispiel 1**

9,04 g (0,04 Mol o-(Epoxy-2,3-propoxy)-diphenyl werden mit 29,2 g (0,4 Mol) tert.-Butylamin fünf Stunden unter Rückfluß erhitzt. Danach wird das Reaktionsgemisch unter vermindertem Druck eingedampft. Der Rückstand wird in 1n-Salzsäure aufgenommen und die saure Lösung mit Diäthyläther extrahiert. Danach wird die saure Lösung mit 10prozentiger Natronlauge alkalisch gemacht. Das sich abscheidende Öl wird mit Diäthyläther extrahiert. Der Ätherextrakt wird über Natriumsulfat getrocknet und eingedampft. Der Rückstand wird unter vermindertem Druck destilliert. Es werden 8,1 g tert.-Butylamino-3-(phenyl-2'-phenoxy)-1-propanol-2 vom Siedepunkt 151 bis 156°C/0,1 mbar erhalten.

Das Hydrochlorid wird durch Einleiten von Chlorwasserstoffgas in eine Lösung von Bipranol in Diäthyläther hergestellt. Beim Stehen in der Kälte erfolgt Kristallisation. Das kristalline Hydrochlorid wird abfiltriert und im Exsikkator getrocknet. F. 123 bis 124°C (im zugeschmolzenen Schmelzpunktröhrchen).

**Beispiel 2**

Herstellungsvorschrift für Tabletten

a) Rezeptur

| | |
|---|---|
| Bipranol - HCl | 75,00g |
| Mikrokristalline Cellulose (Pulver, 50 µm) | 15,75 g |
| Poly-(1-vinyl-2-pyrrolidon) | 5,00 g |
| Hydroxypropylmethylcellulose 2910 | 3,75 g |
| Magnesiumstearat | 0,50 g |
| | 100,00 g |

b) Mischen und Granulieren.

Das Bipranol-HCl wird gegebenenfalls gesiebt, dann werden alle anderen Bestandteile außer Magnesiumstearat in einem Mischer gemischt und ebenfalls im Mischer mit einer geeigneten Menge einer Granulierflüssigkeit (z.B. Wasser oder Isopropanol-Dichlormethan 1:1) befeuchtet. Das feuchte Gemisch wird mit einem geeigneten Sieb gesiebt, im Trockenschrank getrocknet und nochmals gesiebt. Das trockene Granulat wird mit dem Magnesiumstearat im Mischer vermengt.

**Ansprüche**

1. Tert.-Butylamino-3-(phenyl-2'-phenoxy)-1-propanol-2 der Formel

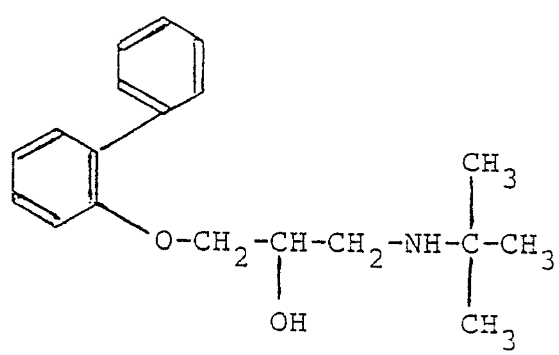

und seine Salze mit Säuren zur Anwendung als Arzneistoff.

2. Verbindung nach Anspruch 1 zur Anwendung bei der Bekämpfung von Herzarrhythmie.

3. Arzneimittel, gekennzeichnet durch einen Gehalt von tert.-Butylamino-3-(phenyl-2'-phenoxy)-1-propanol-2 der Formel

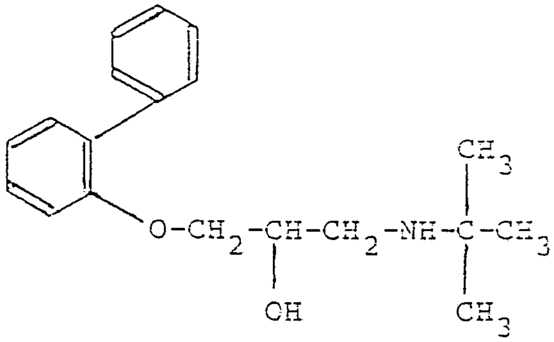

oder dessen Salz mit einer Säure zur Anwendung bei der Bekämpfung von Herzarrhythmie.